# EUROPÄISCHE PATENTSCHRIFT

(11) **EP 2 465 573 B1**
(45) Veröffentlichungstag und Bekanntmachung des Hinweises auf die Patenterteilung: **16.07.2014**
(21) Anmeldenummer: 11191739.9
(22) Anmeldetag: 02.12.2011
(51) Int. Cl.: A61N 1/08

(54) **Implantierbares Gerät**
Implantable device
Appareil implantable

(30) Priorität: 17.12.2010 US 424074 P
(43) Veröffentlichungstag der Anmeldung: 20.06.2012
(73) Patentinhaber: BIOTRONIK SE & Co. KG, 12359 Berlin (DE)
(72) Erfinder: Bartels, Klaus, 10115 Berlin (DE); Büssing, Heinrich, 10317 Berlin (DE); Frenzel, Timo, 12435 Berlin (DE); Kolberg, Gernot, 12161 Berlin (DE); Maxfield, Michelle, 10967 Berlin (DE); Weiss, Ingo, 12435 Berlin (DE)
(74) Vertreter: Lindner-Vogt, Karin L.

(56) Entgegenhaltungen:
- WO-A1-2010/078552
- US-A1- 2007 255 377
- US-A1- 2009 149 920
- US-A1- 2010 318 164

## Beschreibung

Die Erfindung betrifft ein permanent oder temporär implantierbares Gerät mit einem langgestreckten elektrischen Leiter.

Solche Geräte, beispielsweise Elektrodenleitungen für die Elektrostimulation, haben den Nachteil, dass sich ihr elektrischer Leiter in einem Kernspintomografen erwärmen kann, weil die im Kernspintomografen herrschenden wechselnden Magnetfelder in dem elektrischen Leiter nicht unbeachtliche elektrische Ströme induzieren. Deshalb können Herzschrittmacherpatienten heutzutage in der Regel nicht oder nur eingeschränkt in einem Kernspintomografen untersucht werden.

An implantierbaren Herzschrittmachern oder Defibrillatoren sind nämlich typischerweise wenigstens eine Stimulationselektrodenleitung angeschlossen, die an ihrem proximalen, zum Anschluss an den Herzschrittmacher oder Defibrillator vorgesehenen Ende einen standardisierten elektrischen Anschluss aufweist und an ihrem distalen, zur Platzierung im Herzen vorgesehenen Ende einen oder mehrere Elektrodenpole aufweist. Ein solcher Elektrodenpol dient zur Abgabe elektrischer Impulse an das Gewebe (Myokard) des Herzens oder zum Abfühlen elektrischer Felder, um im Rahmen des sogenannten Sensings eine Aktivität eines Herzens abfühlen zu können. Elektrodenpole sind typischerweise in Form eines Rings um die Elektrodenleitung mit einer elektrisch leitenden Oberfläche oder in Form einer Spitzen- oder Tippelektrode am distalen Ende der Elektrodenleitung vorgesehen. Die Elektrodenpole sind über eine oder mehrere elektrische Leitungen mit Kontakten des elektrischen Anschlusses der Elektrodenleitung an deren proximalem Ende elektrisch leitend verbunden. Somit verlaufen zwischen den Kontakten des elektrischen Anschlusses die Elektrodenleitungen an deren proximalen Ende und den Elektrodenpolen am distalen Ende der Elektrodenleitung ein oder mehrere elektrische Leitungen, die einen oder mehrere der Elektrodenpole mit einem oder mehreren der Kontakte elektrisch verbinden. Diese elektrischen Leitungen können einerseits zur Übertragung von Stimulationsimpulsen zu den Elektrodenpolen oder zur Übertragung mittels der Elektrodenpole aufgenommener elektrischer Signale zum proximalen Ende der Elektrodenleitung genutzt werden und werden im Verlauf der weiteren Beschreibung als Funktionsleitung bezeichnet. Solche Funktionsleitungen sind für die Funktionen der jeweiligen Elektrodenleitung erforderliche elektrische Leiter und als solche der Gefahr ausgesetzt, dass in ihnen durch äußere Wechselmagnetfelder elektrische Ströme induziert werden, die beispielsweise zu einer unerwünschten Erwärmung der Funktionsleitungen oder der mit ihr verbundenen Elektrodenpole führen kann. Die WO2010/078552A1, US2010/0318164A1 und US2009/0149920A1 beschreiben Elektrodenleitungen, die gegenüber äußeren Wechselmagnetfeldem unempfindlicher sind.

Der Erfindung, die in Anspruch 1 definiert ist, liegt die Aufgabe zugrunde, ein medizinisches Gerät zu schaffen, welches das zuvor beschriebene Problem löst.

Dabei kann der Funktionsleiter entweder koaxial, mit einem Innenleiter und einem Außenleiter, oder als Seil aufgebaut sein.

Bei einem koaxialen Aufbau wird diese Aufgabe durch ein temporär oder permanent implantierbares medizinisches Gerät mit
- mindestens einer langgestreckten ersten elektrischen Leitung mit einem Funktionsleiter, einen Innenleiter und einen Außenleiter enthält und der der mit einem Funktionselektrodenpol zur Abgabe von Therapiesignalen oder zur Erfassung von Diagnosesignalen verbunden ist, und mit
- mindestens einem mit dem ersten elektrischen Leiter gekoppelten und in einer gemeinsamen isolierenden Ummantelung geführten zweiten elektrischen Leiter, wobei eine ohmsche Kopplung zwischen dem ersten und dem zweiten elektrischen Leiter ausgebildet ist, im ersten Leiter geführte elektromagnetische Radiofrequenz-Wellen zumindest teilweise in den zweiten elektrischen Leiter einzukoppeln.

Das erfindungsgemäße medizinische Gerät erzielt eine Verringerung der unerwünschten Erwärmung der Funktionsleitungen oder der mit ihr verbundenen Elektrodenpole, die durch elektrische Ströme verursacht werden, welche äußere Wechselmagnetfelder im Funktionsleiter der elektrischen Leitung induzieren können. Auf diese Weise kann eine unerwünschte Erwärmung von Körpergewebe im implantierten Zustand reduziert, zumindest teilweise auf andere Gewebebereiche verlagert oder sogar vollständig vermieden werden. Dies gelingt erfindungsgemäß mit Hilfe eines zweiten elektrischen Leiters, der im Rahmen dieser Anmeldung auch als Zusatzleiter bezeichnet wird, und mit Hilfe einer ohmschen Kopplung zwischen dem ersten und dem zweiten elektrischen Leiter, die ausgebildet ist, im ersten Leiter geführte elektromagnetische Radiofrequenz-Wellen zumindest teilweise in den zweiten elektrischen Leiter einzukoppeln.

Nachfolgend werden Ausführungsbeispiele des medizinischen Geräts der vorliegenden Erfindung erläutert. Die zusätzlichen Merkmale der einzelnen Ausführungsbeispiele können miteinander zur Bildung weiterer Ausführungsformen des medizinischen Geräts kombiniert werden, soweit sie nicht ausdrücklich als einander ausschließende Alternativen beschrieben sind.

Die langgestreckte elektrische Leitung ist in bevorzugten Ausführungsbeispielen eine temporär oder permanent implantierbare Elektrodenleitung zur Verbindung eines oder mehrerer Funktions-Elektrodenpole mit einem Steuergerät, wie beispielsweise dem Steuergerät eines implantierbaren Herzschrittmachers oder eines implantierbaren Defibrillators. Die langgestreckte Leitung bildet jedoch als solche schon ein medizinisches Gerät im Sinne der vorliegenden Beschreibung.

Die Kopplung zwischen dem Funktionsleiter und dem zweiten elektrischen Leiter (Zusatzleiter) ist eine ohmsche Kopplung.

Die Kopplung ist in einer Ausführungsform an nur einer Stelle der Längserstreckung des Funktionsleiters vorgesehen. In einer Variante sind mehrere Kopplungsstellen entlang der Längserstreckung des Funktionsleiters vorhanden. Dabei kann an allen Kopplungsstellen dieselbe Kopplungsform zum Einsatz kommen. Alternativ kommen an unterschiedlichen Stellen unterschiedliche Kopplungsformen zum Einsatz.

Es können bei dem medizinischen Gerät zwei oder mehr Zusatzleiter mit dem Funktionsleiter gekoppelt sein. Bei einer Ausführungsform ist der Funktionsleiter über seine Längserstreckung zwischen seinem proximalen Ende und dem Funktionselektrodenpol mit einer Vielzahl Zusatzleiter gekoppelt. Das bedeutet, dass der Funktionsleiter mit jedem der betreffenden Zusatzleiter über eine individuelle Kopplung gekoppelt ist. Dies kann so ausgeführt sein, dass der Funktionsleiter und der betreffende Zusatzleiter in einer jeweiligen der Vielzahl miteinander in Reihe verbundener Parallelschaltungen mit einander verknüpft sind. Es kann aber auch so ausgeführt sein, dass beispielsweise zwei Zusatzleiter vorhanden sind.

Diese Ausführungsform kann in unterschiedlicher Weise fortgebildet werden. In einer Variante dieser Ausführungsform hat mindestens ein zweiter der Zusatzleiter eine der Länge des Funktionsleiters zumindest ungefähr entsprechende Längserstreckung. In einer anderen Variante sind die Zusatzleiter jeweils durch ein kurzes Leiterstück gebildet, so dass eine Vielzahl kurzer Leiterstücke als jeweilige Zusatzleiter Verwendung finden, die jeweils individuell mit dem Funktionsleiter gekoppelt sind. Diese kurzen Leiterstücke haben also jeweils eine deutlich geringere Längserstreckung als der Funktionsleiter selbst. Sie werden vorzugsweise so gestaltet, dass sie über ihre Längserstreckung eine möglichst starke Kopplung mit dem Funktionsleiter erzielen.

Die verschiedenen Zusatzleiter sind in alternativen Fortbildungen dieser Ausführungsform entweder miteinander gekoppelt oder voneinander entkoppelt. Ein Beispiel einer resistiven Kopplung von Zusatzleitern untereinander sieht eine elektrische Serienschaltung von mindestens zwei der kurzen Zusatzleiter vor. Eine solche Kopplung, beispielsweise im Falle zweier Zusatzleitungen, ist bei einem Ausführungsbeispiel an mehreren Stellen der gemeinsamen Längserstreckung der Zusatzleiter vorgesehen.

Alternativ sind mehrere Zusatzleitungen jeweils parallel mit dem Funktionsleiter gekoppelt.

Hinsichtlich der Anzahl miteinander gekoppelter oder entkoppelter Zusatzleiter besteht eine Auswahlmöglichkeit, je nach den Bedürfnissen einer jeweiligen Anwendung.

In einer Ausführungsform ist der Zusatzleiter oder ist mindestens einer der Vielzahl Zusatzleiter zusätzlich mit dem Funktionselektrodenpol oder einem davon verschiedenen zweiten Funktionselektrodenpol gekoppelt.

Eine Kopplung mit einem zweiten Funktionsleiter der elektrischen Leitung und/oder einem zweiten Funktionselektrodenpol ist sinnvoll, um mit Hilfe eines und desselben Zusatzleiters zusätzlich einen solchen zweiten Funktionsleiter oder einen mit ihm verbundenen zweiten Funktionselektrodenpol vor unerwünschter Erwärmung zu schützen.

Der Zusatzleiter ist in zueinander alternativen Ausführungsformen unisoliert, teilweise isoliert oder isoliert. Der Zusatzleiter wird vorzugsweise so gestaltet, dass er im Hinblick auf den Energieübertrag in das Körpergewebe optimal mit dem Körpergewebe gekoppelt ist. Hierzu kann in einer Ausführungsform mindestens ein in seiner Funktion von einem Funktionselektrodenpol verschiedener zweiter Elektrodenpol mit dem Zusatzleiter verbunden sein. Der zweite Elektrodenpol, der auch als Opferelektrodenpol bezeichnet werden kann, hat also keine diagnostische oder therapeutische Funktion, sondern dient im implantierten Zustand zur den Funktionselektrodenpol und/oder die Funktionsleitung entlastenden Einkopplung von Energie in das umgebende Körpergewebe an mindestens einem vom Funktionselektrodenpol verschiedenen Ort.

Der Zusatzleiter kann in einem solchen Ausführungsbeispiel zum Gewebe hin elektrisch isoliert sein, beispielsweise durch eine dielektrische Schicht auf dem Leitermaterial im Bereich des Funktionselektrodenpols oder des zweiten Elektrodenpols. Auf diese Weise wird im implantierten Zustand eine galvanische Kopplung zum umgebenden Köpergewebe verhindert, jedoch beispielsweise eine kapazitive oder induktive Kopplung ermöglicht.

Bei einer solchen Ausführungsform ist eine galvanische Kopplung des Zusatzleiters mit dem Funktionsleiter in mindestens einem vom Elektrodenpol verschiedenen Längsabschnitt vorgesehen. Eine solche galvanische Kopplung kann insbesondere verteilt an verschiedenen Stellen über die gemeinsame Längserstreckung des Funktionsleiters und des Zusatzleiters hinweg angeordnet sein. Dies kann beispielsweise mit Hilfe mehrerer galvanischer Schleifkontakte erreicht werden, die über die gemeinsame Längserstreckung hin verteilt sind. Vorzugsweise lässt die galvanische Kopplung jedoch eine mechanische Beweglichkeit von Funktionsleiter und Zusatzleiter zueinander zu. Dies gelingt ein einer bevorzugten Ausführungsform mit Hilfe eines oder mehrerer Schleifkontakte. Zur Verbesserung der Kopplung ohne Einschränkung der Beweglichkeit kann der Zusatzleiter leitfähige Whisker haben.

Sind bei der vorstehend beschriebenen Ausführungsform, die eine galvanische Kopplung zwischen Funktionsleiter und Zusatzleiter vorsieht, mehrere Funktionsleiter mit einem oder mehreren jeweiligen Zusatzleitern gekoppelt, sollten die nicht isolierenden Abschnitte unterschiedlicher Zusatzleiter vor wechselseitigem Kurzschluss gesichert sein, sich also nicht gegenseitig berühren können.

Bei einer Ausführungsform ist der Zusatzleiter hohlwendelförmig und umgibt den Innenleiter des Funktionsleiters. Er kann beispielsweise koaxial um den ersten Leiter gewendelt sein. Alternativ umgibt der Funktionsleiter den Zusatzleiter, beispielsweise ebenfalls in einer koaxialen Wicklung. Eine andere Alternative ist eine koradiale Anordnung von Funktionsleiter und Zusatzleiter, bei der in einer Variante ein Außenleiter des Funktionsleiters und der Zusatzleiter auf einer gemeinsamen gedachten Zylinder-Mantelfläche um einen Innenleiter des Funktionsleiters winden, wobei der Außenleiter des Funktionsleiters vom Zusatzleiter elektrisch isoliert ist. In einer alternativen Variante windet sich ein Außenleiter des Funktionsleiters wendelförmig um den Zusatzleiter und einen Innenleiter des Funktionsleiters, wobei der Innenleiter und der Zusatzleiter in einer gedachten gemeinsamen Zylinder-Mantelfläche jeweils hohlwendelförmig geführt sind und der Außenleiter vom zweiten elektrischen Leiter elektrisch isoliert ist.

Die Ausführungsbeispiele mit induktiver oder kapazitiver Kopplung sind nicht Teil der Erfindung.

Weitere Ausführungsbeispiele des medizinischen Geräts werden nachfolgend anhand der Figuren erläutert. Es zeigen:
- Fig. 1: Ausführungsbeispiele medizinischer Geräte in Form eines Herzschrittmachers und einer daran angeschlossenen Elektrodenleitung;
- Fig. 2: ein Ersatzschaltbild eines Ausführungsbeispiels einer Elektrodenleitung, bei der eine induktive Kopplung zwischen einem Funktionsleiter und einem Zusatzleiter realisiert ist;
- Fig. 3: ein Ersatzschaltbild eines Ausführungsbeispiels einer Elektrodenleitung, bei der parallel zu den Drähten einer Außenleiterwendel ein oder mehrere Zusatzleiter geführt werden, die an ihren Enden je einen Elektrodenpol haben.
- Fig. 4: ein Ersatzschaltbild eines Ausführungsbeispiels einer Elektrodenleitung, bei der eine kapazitive Kopplung zweier Zusatzleiter mit den Funktionsleitem realisiert ist;
- Fig. 5: eine schematische Längsschnittansicht einer Elektrodenleitung, bei der eine galvanische Kopplung zwischen einem Funktionsleiter und einem Zusatzleiter über Schleifkontakte realisiert ist;
- Fig. 6: eine Längsschnittsansicht einer Elektrodenleitung mit Ableitringen;
- Fig. 7: ein Ersatzschaltbild eines anderen Ausführungsbeispiels einer Elektrodenleitung, bei der parallel zu den Leitern einer Außenleiterwendel ein oder mehrere Zusatzleiter geführt werden.

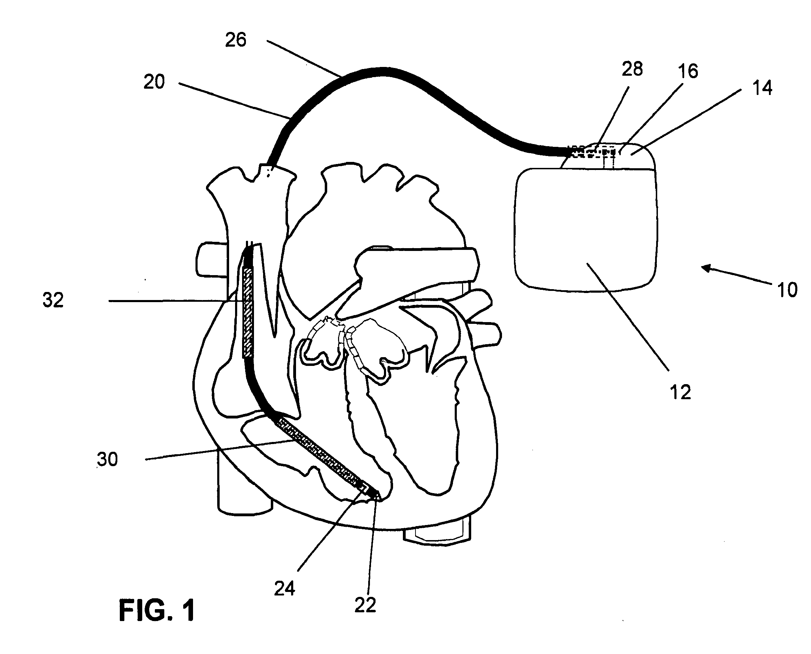

Fig. 1 zeigt als Beispiele implantierbarer medizinischer Geräte einen implantierbaren Herzstimulator 10 und eine daran angeschlossene implantierbare Elektrodenleitung 20.

Der implantierbare Herzstimulator 10 kann ein Herzschrittmacher oder ein Kardioverter/Defibrillator (ICD) sein. Im dargestellten Ausführungsbeispiel ist der Herzstimulator 10 ein ventrikulärer Herzschrittmacher und Defibrillator. Andere bekannte Herzstimulatoren sind Zweikammerherzschrittmacher zur Stimulation des rechten Atriums und des rechten Ventrikels oder biventrikuläre Herzschrittmacher, die zusätzlich zum rechten Ventrikel auch den linken Ventrikel stimulieren können.

Derartige Stimulatoren besitzen typischerweise ein Gehäuse 12, das im Regelfall aus Metall besteht und somit elektrisch leitend ist und als großflächiger Elektrodenpol dienen kann. An der Außenseite des Gehäuses 12 ist typischerweise ein Anschlussgehäuse 14 befestigt, das auch als Header bezeichnet wird. Ein derartiger Header weist typischerweise Kontaktbuchsen zur Aufnahme von Steckkontakten auf. Die Kontaktbuchsen besitzen elektrische Kontakte 16, die über entsprechende Leiter mit einer in dem Gehäuse 12 des Herzstimulators 10 angeordneten Elektronik verbunden sind.

Die Elektrodenleitung 20 stellt im Sinne dieser Erfindung ebenfalls ein implantierbares medizinisches Gerät dar. Am distalen Ende der Elektrodenleitung 20 sind in an sich bekannter Manier Elektrodenpole in Form einer Spitzen- oder Tip-Elektrode 22 und einer in deren Nähe angeordneten Ringelektrode 24 angeordnet. Die Elektrodenpole 22 und 24 sind so ausgebildet, dass sie je nach Funktion eines Herzstimulators, an den die Elektrodenleitung 20 angeschlossen ist, zum Abfühlen elektrischer Potenziale des Herzgewebes (Myokards) dienen oder zur Abgabe elektrischer Signale, beispielsweise zur Abgabe von Stimulationsimpulsen an das sie umgebende Herzgewebe, ausgebildet sind. Fig. 1 zeigt, wie sich die Elektrodenpole, also die Tip-Elektrode 22 und die Ringelektrode 24, im Anwendungsfall die Elektrodenleitung 20, im Apex eines rechten Ventrikels eines Herzens befinden.

Sowohl die Tip-Elektrode 22 als auch die Ringelektrode 24 sind über jeweils wenigstens einen elektrischen Leiter 26 mit einem Steckkontakt 28 am proximalen Ende der Elektrodenleitung 20 elektrisch verbunden. Der Steckkontakt 28 besitzt elektrische Kontakte, die mit den elektrischen Kontakten 16 der Kontaktbuchse im Anschlussgehäuse 14 des implantierbaren Herzstimulators korrespondieren.

Wie weiter unten näher erläutert wird, können die elektrischen Leiter 26 in der Elektrodenleitung 20 in unterschiedlichen Längsabschnitten als annähernd gestreckte Seilzugleiter oder als helixförmig gewendelte Leiter ausgebildet sein. Solche Leiter, die funktionale Elektrodenpole mit elektrischen Kontakten des Steckkontaktes am proximalen Ende der Elektrodenleitung 20 elektrisch leitend verbinden, werden im Rahmen dieses Textes auch als Funktionsleiter bezeichnet, da sie beispielsweise der Therapie dienende elektrische Signale von Steckkontakt zum jeweiligen Elektrodenpol übertragen oder abgefühlte elektrische Potentiale repräsentierende Signale vom jeweiligen Elektrodenpol zum Steckkontakt führen und somit der elementaren Funktion des medizinischen Gerätes dienen.

Die elektrischen Funktionsleiter 26, die die Elektrodenpole 22 bzw. 24 mit den elektrischen Kontakten des Steckers 28 der Elektrodenleitung 20 verbinden, sind über den größten Teil ihrer Länge von einer isolierenden Hülle umgeben, so dass ein elektrischer Kontakt zum Gewebe des Herzens gezielt über die Elektrodenpole zustande kommt.

Neben den Elektrodenpolen 22 und 24, die typischerweise der (in diesem Fall ventrikulären) Stimulation des Herzgewebes dienen, weist die Elektrodenleitung 20 auch noch zwei großflächigere Elektrodenpole 30 und 32 auf, die als Defibrillationselektroden dienen und von wenigstens einem blank liegendem helixartig gewendelten Draht gebildet sind.

Es sei darauf hingewiesen, dass die Erfindung im Rahmen dieses Ausführungsbeispiels anhand eines rechtsventrikulären Herzschrittmachers und Defibrillators erläutert wird. Als medizinisches Gerät im Sinne der Erfindung kann grundsätzlich aber beispielsweise auch eine Ablationselektrodenleitung dienen, die im Anwendungsfall ebenfalls bis ins Herz eines Patienten hineinragt und die von einem außerhalb des Patienten angeordneten Gerät gesteuert wird und hierzu mit diesem verbunden ist. Weiterhin können solche Elektrodenleitungen unter fachüblicher Anpassung an die speziellen Erfordernisse des jeweiligen Anwendungsgebiets auch zur Stimulation und Ableitung von Signalen an Nerven, Gehirn, und anderen Organen oder für die Zuleitung von implantierbaren Sensoren Verwendung finden.

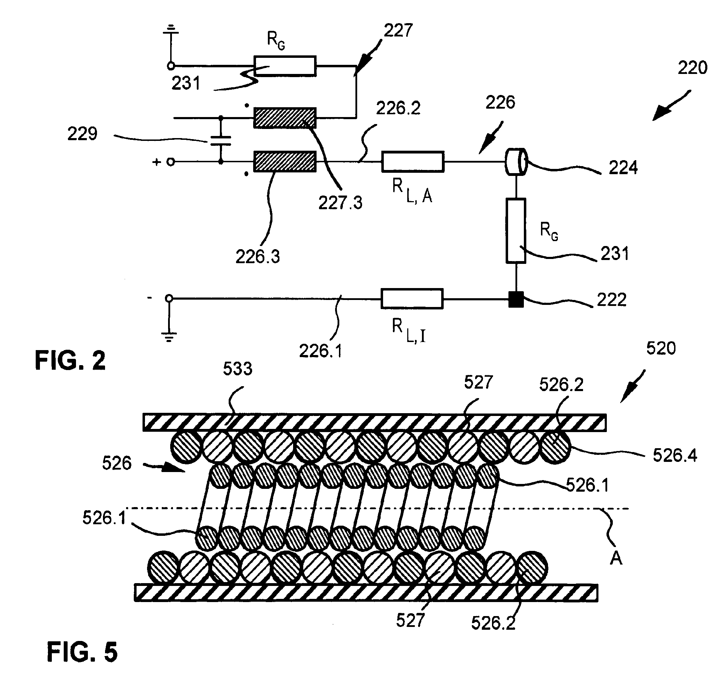

Nachfolgend werden anhand von Ersatzschaltbildern der Fig. 2 bis 4 unterschiedliche Ausführungsbeispiele der Elektrodenleitung 26 beschrieben. Es sei angemerkt, dass in den Figuren 2 bis 5 Bezugszeichen verwendet sind, die den Zusammenhang der dort dargestellten Strukturelemente einer Elektrodenleitung mit dem Ausführungsbeispiel der Fig. 1 verdeutlichen sollen. Dazu enthalten letzten beiden Ziffern eines auch in Fig. 1 dargestellten Strukturelements jeweils das in Fig. 1 verwendete Bezugszeichen. Dies impliziert jedoch nur eine funktionelle Entsprechung und soll, soweit nichts Anderes gesagt ist, nicht etwa bedeuten, dass es sich um identische Strukturelemente handelt.

Fig. 2 zeigt ein Ersatzschaltbild eines Ausführungsbeispiels einer Elektrodenleitung 220, bei der eine induktive Kopplung zwischen einem Funktionsleiter 226 und einem Zusatzleiter 227 realisiert ist, im implantierten Zustand.

Ein Funktionsleiter 226 hat einen Innenleiter 226.1 und einen Außenleiter 226.2, die proximal mit einer Spannungsversorgung eines in diesem Ersatzschaltbild der Einfachheit halber auf seine Funktion als Spannungsquelle reduzierten Steuergeräts verbunden sind. Der Innenleiter 226.1 ist distal mit einem Tip-Elektrodenpol 222 verbunden. Der Außenleiter 226.2 ist mit einem Ring-Elektrodenpol 224 verbunden. Beide Elektrodenpole 222 und 224 sind über ein Körpergewebe, das durch einen ohmschen Gewebewiderstand 231 repräsentiert ist, verbunden.

Ein Zusatzleiter 227, der im Rahmen dieser Beschreibung auch als zweiter elektrischer Leiter bezeichnet wird, ist mit dem Außenleiter 226.1 induktiv und kapazitiv gekoppelt. Eine induktive Kopplung 226.3, 227.3 wird mittels einer wendelförmigen, bevorzugt aber nicht zwingend gleichsinnigen Wicklung der beiden gekoppelten Leiter, also des Außenleiters 226.2 und des Zusatzleiters 227 erzielt, die dadurch im Ersatzschaltbild der Fig. 2 gekoppelte Induktivitäten 226.3 und 227.3 bilden. Eine zusätzliche kapazitive Kopplung 229 der beiden Leiter 226.2 und 227 ergibt sich dadurch, dass die beiden Leiter 226.2 und 227 koradial nebeneinander verlaufen und elektrisch von einander isoliert sind. Durch den Abstand der beiden Leiter 226.2 und 227 in ihrer gemeinsamen Wicklung voneinander und/oder durch die Wahl der relativen Permittivität des Isolators zwischen ihnen kann Kapazität 229 beeinflusst werden.

Der Zusatzleiter 227 ist mit dem Körpergewebe 231 über einen zusätzlichen, hier nicht dargestellten Elektrodenpol verbunden, so dass über die induktive und kapazitive Kopplung in den Zusatzleiter 227 eingespeiste Energie in das Körpergewebe abgeführt werden kann.

Mit dieser Elektrodenleitung gelingt eine Verringerung der unerwünschten Erwärmung der Funktionsleitung 226 oder der mit ihr verbundenen Elektrodenpole 222 und 224, die durch elektrische Ströme verursacht werden, welche starke äußere Wechselmagnetfelder wie sie beispielsweise von Magnetresonanz-Geräten erzeugt werden, im Funktionsleiter der Elektrodenleitung induzieren können. Auf diese Weise kann eine unerwünschte Erwärmung von Körpergewebe im Bereich der Elektroden 222 und 224 im implantierten Zustand reduziert und zumindest teilweise auf andere Gewebebereiche verlagert oder sogar vollständig vermieden werden.

Dies gelingt mit Hilfe des Zusatzleiters 227, und mit Hilfe der beschriebenen Kopplung zwischen dem Funktionsleiter und dem Zusatzleiter, die ausgebildet ist, im ersten Leiter geführte elektromagnetische Radiofrequenz-Wellen zumindest teilweise in den zweiten elektrischen Leiter einzukoppeln.

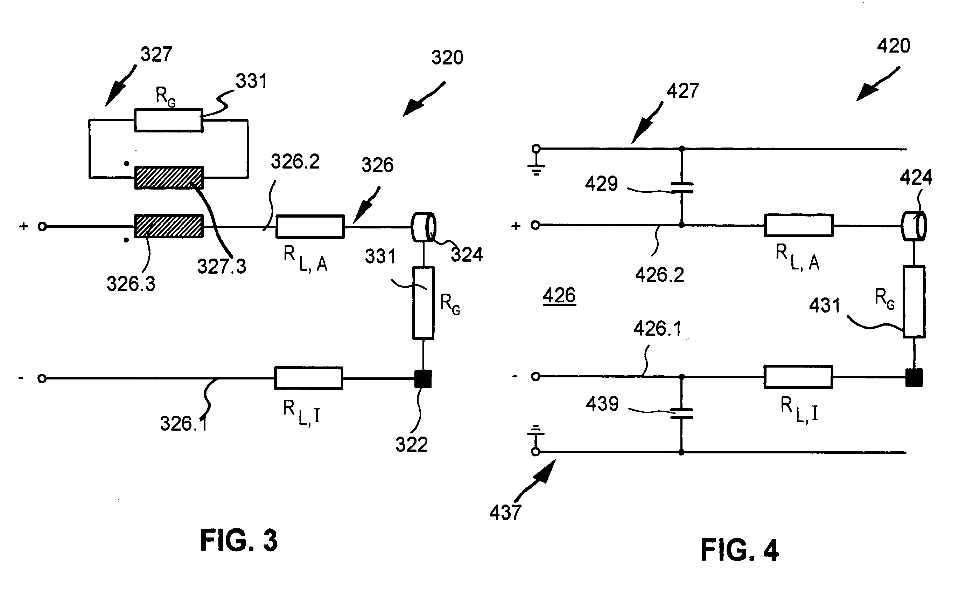

Fig. 3 zeigt ein Ersatzschaltbild eines anderen Ausführungsbeispiels einer Elektrodenleitung 320 im implantierten Zustand, bei der parallel zu den Leitern einer Außenleiterwendel 326.2 ein oder mehrere, bevorzugt wendelförmige Zusatzleiter 327 geführt werden, die an ihren Enden je einen ringförmigen Elektrodenpol (nicht dargestellt) haben. Im Bereich zwischen 326.3 und 327.3 ist die Wendelförmige Ausführung besonders bevorzugt.

Ein Funktionsleiter 326 hat einen Innenleiter 326.1 und einen Außenleiter 326.2. Der Innenleiter 326.1 ist distal mit einem Tip-Elektrodenpol 322 verbunden. Der Außenleiter 326.2 ist mit einem Ring-Elektrodenpol 324 verbunden. Beide Elektrodenpole 322 und 324 sind über ein Körpergewebe, das durch einen ohmschen Gewebewiderstand 331 repräsentiert ist, verbunden.

Ein Zusatzleiter 327 ist mit dem Außenleiter 326.2 induktiv gekoppelt. Die von dem Zusatzleiter 327 über die induktive Kopplung aufgenommene Energie wird von einem (nicht dargestellten) ringförmigen Elektrodenpol ins Körpergewebe 331 abgeführt. Dabei wird die praktisch immer vorhandene kapazitive Kopplung nicht eingegangen. In Fig. 3 sind die Elektrodenpole so dargestellt, dass der kürzeste Weg für den Strom über die Ableitpole geht, wohingegen in Fig. 2 der Stromkreis über Rg und die Fernkugel geschlossen wird. Alternativ kann auch nur ein Ableitpol vorhanden sein, dann wird der Stromkreis über eine Kapazitive Kopplung geschlossen. Auch muss eine Masse nicht technisch implementiert werden, diese kann über das Potential der Fernkugel realisiert sein. Die Fernkugel ist dabei durch ein Potential im Unendlichen definiert, wobei elektrische Felder schnell abklingen.

Die Elektrodenleitung 320 hat dieselben die Vorteile, die im Zusammenhang der Beschreibung der Elektrodenleitung 220 der Fig. 2 erläutert wurden.

Fig. 4 zeigt ein Ersatzschaltbild eines weiteren Ausführungsbeispiels einer Elektrodenleitung 420, bei der eine kapazitive Kopplung zweier Zusatzleiter mit den Funktionsleitern realisiert ist.

Ein Funktionsleiter 426 hat einen Innenleiter 426.1 und einen Außenleiter 426.2. Der Innenleiter 426.1 ist distal mit einem Tip-Elektrodenpol 422 verbunden. Der Außenleiter 426.2 ist mit einem Ring-Elektrodenpol 424 verbunden. Beide Elektrodenpole 422 und 424 sind über ein Körpergewebe, das durch einen ohmschen Gewebewiderstand 431 repräsentiert ist, verbunden.

In diesem Ausführungsbeispiel sind zwei Zusatzleiter 427 und 437 vorhanden. Der erste Zusatzleiter 427 ist mit dem Außenleiter 426.2 kapazitiv gekoppelt. Der zweite Zusatzleiter 437 ist mit dem Innenleiter 426.1 kapazitiv gekoppelt.

Die Elektrodenleitung 420 hat dieselben die Vorteile, die im Zusammenhang der Beschreibung der Elektrodenleitung 220 der Fig. 2 erläutert wurden.

Fig. 5 zeigt eine schematische Längsschnittansicht einer Elektrodenleitung 520, bei der eine galvanische Kopplung zwischen einem Funktionsleiter und einem Zusatzleiter über Schleifkontakte realisiert ist.

Die Elektrodenleitung 520 enthält in einer Ummantelung 533 einen Funktionsleiter 526, der einen wendelförmig gewickelten Innenleiter 526.1 und einen mit größerem Radius ebenfalls wendelförmig gewickelten Außenleiter 526.2 umfasst. Der Außenleiter ist mit einer Isolierung 526.4 versehen, während der Innenleiter im hier dargestellten Längsabschnitt unisoliert ist. Der Außenleiter 526.2 ist mit einem Zusatzleiter 527 koradial bezüglich einer Mittelachse A der zylindrischen Elektrodenleitung 520 gewickelt. Der Zusatzleiter ist im hier dargestellten Längsabschnitt unisoliert. Der Innenleiter liegt mit seinen Außenflächen an den Innenflächen des Außenleiters 526.2 und des Zusatzleiters 527 an. Auf diese Weise entsteht an den gemeinsamen Berührungsflächen des unisolierten Innenleiters 526.1 und des unisolierten Zusatzleiters 527 eine galvanische Kopplung in Form eines Schleifkontaktes. Der Innenleiter kann sich also gegen den Zusatzleiter bewegen, ohne dass der galvanische Kontakt verloren geht.

Der Zusatzleiter 527 ist also zum Gewebe hin durch die Ummantelung 533 isoliert, nicht aber zum therapeutisch oder diagnostisch genutzten Funktionsleiter 526. Das heißt, es kann über die Leiterlänge verteilt galvanisch Kontakt bestehen. Trotzdem sind bei dem hier realisierten verteilten Schleifkontakt der Funktionsleiter 526 und sein Zusatzleiter 527 mechanisch zueinander beweglich.

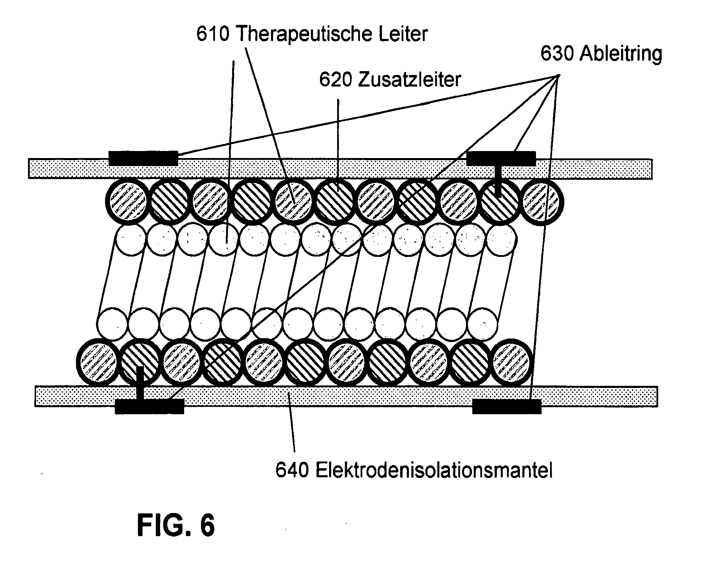

Fig. 6 zeigt eine Längsschnittsansicht einer Elektrodenleitung mit Ableitringen 630, die einen elektrischen Kontakt zum Zusatzleiter 620 aufweisen und teilweise aus dem Elektrodenisolationsmantel 640 herausragen. Auch gezeigt werden die Therapeutischen Leiter 610.

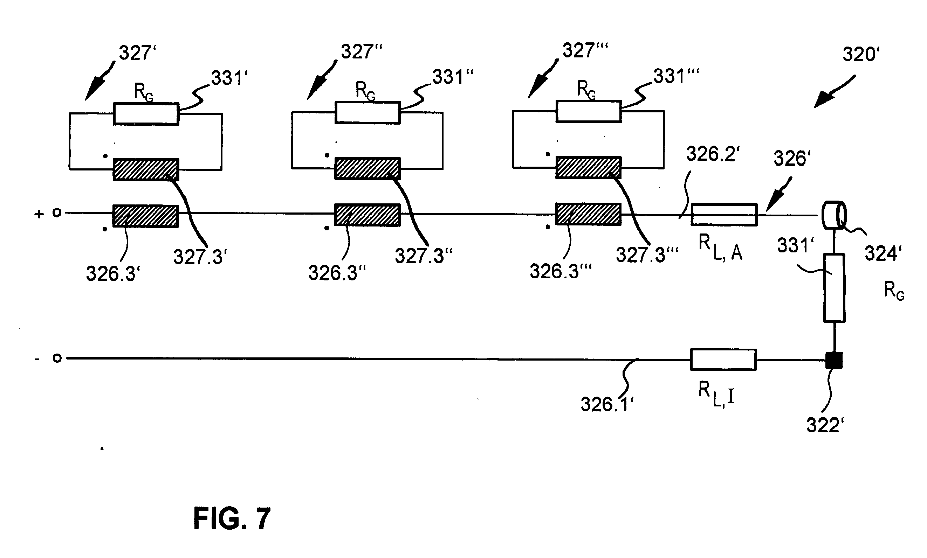

Fig. 7 Zeigt ein Ersatzschaltbild eines anderen Ausführungsbeispiels einer Elektrodenleitung 320' im implantierten Zustand, ähnlich der Fig. 3, allerdings zeigt Fig. 7 eine Gruppe gewendelter Zusatzleiter 327', 327", 327"' mit 2 Ableitpolen, die im bestimmen Abständen wiederholt entlang der Leitung eingebaut werden. Die Zusatzleiter selbst sind aber nicht in Reihe geschaltet. Abgesehen von den zur Unterscheidung gedachten " ' " bezeichnen die Bezugszeichen die gleichen Elemente wie in Fig. 3.

Es sei erwähnt, dass in allen vorstehend beschriebenen Ausführungsbeispielen wahlweise der Funktionsleiter oder der Zusatzleiter eine höhere Leitfähigkeit als der jeweils andere dieser beiden Leiter aufweisen kann.

## Patentansprüche

1. Temporär oder permanent implantierbares medizinisches Gerät mit
- mindestens einer langgestreckten ersten elektrischen Leitung (20) mit einem Funktionsleiter (26, 226, 326, 426, 526), der mit einem Funktionselektrodenpol (22, 24, 30, 32, 222, 224, 322, 324, 422, 424) zur Abgabe von Therapiesignalen oder zur Erfassung von Diagnosesignalen verbunden ist, und mit
- mindestens einem mit dem Funktionsleiter (26, 226, 326, 426, 526) gekoppelten und mit ihm in einer gemeinsamen isolierenden Ummantelung (533) geführten zweiten elektrischen Leiter (227, 327, 327', 327", 327"', 427, 437, 527, 620), wobei eine Kopplung zwischen dem Funktionsleiter (26, 226, 326, 426, 526) und dem zweiten elektrischen Leiter (227, 327, 327', 327", 327"', 427, 437, 527, 620) ausgebildet ist, im Funktionsleiter (26, 226, 326, 426, 526) geführte elektromagnetische Radiofrequenz-Wellen zumindest teilweise in den zweiten elektrischen Leiter (227, 327, 327', 327", 327"', 427, 437, 527, 620) einzukoppeln, wobei der Funktionsleiter (26, 226, 326, 426, 526) und der zweite elektrische Leiter (227, 327, 327', 327", 327"', 427, 437, 527, 620) durch eine ohmsche Kopplung gekoppelt sind, und dadurch charakterisiert, dass der Funktionsleiter einen Innenleiter und Außenleiter aufweist,
wobei entweder
- der zweite elektrische Leiter (227, 327, 327', 327", 327"', 427, 437, 527, 620) und ein Außenleiter des Funktionsleiters (26, 226, 326, 426, 526) sich wendelförmig auf einer gemeinsamen gedachten Zylinder-Mantelfläche um den Innenleiter des ersten elektrischen Leiters winden, wobei der Außenleiter des Funktionsleiters (26, 226, 326, 426, 526) vom zweiten elektrischen Leiter (227, 327, 327', 327", 327"', 427, 437, 527, 620 elektrisch isoliert ist, oder
- sich ein Außenleiter des Funktionsleiters (26, 226, 326, 426, 526) wendelförmig um den zweiten elektrische Leiter (227, 327, 327', 327", 327"', 427, 437, 527, 620 und einen Innenleiter des Funktionsleiters (26, 226, 326, 426, 526) windet, wobei der Innenleiter und der zweite elektrische Leiter (227, 327, 327', 327", 327"', 427, 437, 527, 620 in einer gemeinsamen gedachten Zylinder-Mantelfläche jeweils hohlwendelförmig geführt sind und der Außenleiter vom zweiten elektrischen Leiter (227, 327, 327', 327", 327"', 427, 437, 527, 620 elektrisch isoliert ist.

2. Medizinisches Gerät nach einem der vorstehenden Ansprüche, bei dem der Funktionsleiter (26, 226, 326, 426, 526) über seine Längserstreckung zwischen seinem proximalen Ende und dem Funktionselektrodenpol (22, 24, 30 ,32, 222, 224, 322, 324, 422, 424) mit einer Vielzahl zweiter elektrischer Leiter (227, 327, 327', 327", 327"', 427, 437, 527, 620) gekoppelt ist.

3. Medizinisches Gerät nach Anspruch 2, bei dem mindestens zwei der zweiten elektrischen Leiter (227, 327, 327', 327", 327"', 427, 437, 527, 620) miteinander in Form einer elektrischen Serienschaltung gekoppelt sind.

4. Medizinisches Gerät nach Anspruch 2, bei dem mindestens zwei der zweiten elektrischen Leiter (227, 327, 327', 327", 327"', 427, 437, 527, 620) miteinander in Form einer elektrischen Parallelschaltung gekoppelt sind.

5. Medizinisches Gerät nach einem der vorstehenden Ansprüche, bei dem der zweite elektrische Leiter (227, 327, 327', 327", 327"', 427, 437, 527, 620) zusätzlich mit dem Funktionselektrodenpol durch eine weitere Kopplung gekoppelt ist.

6. Medizinisches Gerät nach einem der vorstehenden Ansprüche, bei dem der zweite elektrische Leiter mit einem von dem Funktionselektrodenpol (22, 24, 30 ,32, 222, 224, 322, 324, 422, 424) verschiedenen zweiten Elektrodenpol verbunden ist.

7. Medizinisches Gerät nach Anspruch 6, bei dem der zweite Elektrodenpol mit einer dielektrischen Schicht bedeckt ist.

8. Medizinisches Gerät nach einem der vorstehenden Ansprüche, bei dem der zweite elektrische Leiter (227, 327, 327', 327", 327"', 427, 437, 527, 620) hohlwendelförmig ist und den Innenleiter des Funktionsleiters (26, 226, 326, 426, 526) umgibt.

9. Medizinisches Gerät nach einem der vorstehenden Ansprüche, bei dem der Innenleiter des Funktionsleiters (26, 226, 326, 426, 526) mit dem zweiten elektrischen Leiter (227, 327, 327', 327", 327"', 427, 437, 527, 620) durch mindestens einen Schleifkontakt gekoppelt ist.

## Claims

1. A temporarily or permanently implantable medical device, comprising:
- at least one elongated first electrical conductor (20) having a functional conductor (26, 226, 326, 426, 526), which is connected to a function electrode pole (22, 24, 30, 32, 222, 224, 322, 324, 422, 424) for delivering therapeutic signals or for detecting diagnostic signals; and
- at least one second electrical conductor (227, 327, 327', 327", 327"', 427, 437, 527, 620), which is coupled to the functional conductor (26, 226, 326, 426, 526) and is guided together therewith in a shared insulating sheath (533), a coupling between the functional conductor (26, 226, 326, 426, 526) and the second electrical conductor (227, 327, 327', 327", 327"', 427, 437, 527, 620) being designed to incouple at least some of the electromagnetic radio frequency waves that are conducted in the functional conductor (26, 226, 326, 426, 526) into the second electrical conductor (227, 327, 327', 327", 327"', 427, 437, 527, 620), the functional conductor (26, 226, 326, 426, 526) and the second electrical conductor (227, 327, 327', 327", 327"', 427, 437, 527, 620) being coupled by a resistance coupling,
**characterized in that** the functional conductor comprises an inner conductor and an outer conductor, wherein either
- the second electrical conductor (227, 327, 327', 327", 327"', 427, 437, 527, 620) and the outer conductor of the functional conductor (26, 226, 326, 426, 526) wind spirally around the inner conductor of the first electrical conductor on a shared imaginary lateral cylinder surface, the outer conductor of the functional conductor (26, 226, 326, 426, 526) being electrically insulated with respect to the second electrical conductor (227, 327, 327', 327", 327"', 427, 437, 527, 620), or
- the outer conductor of the functional conductor (26, 226, 326, 426, 526) winds helically around the second electrical conductor (227, 327, 327', 327", 327"', 427, 437, 527, 620) and the inner conductor of the functional conductor (26, 226, 326, 426, 526), the inner conductor and the second electrical conductor (227, 327, 327', 327", 327"', 427, 437, 527, 620) being guided in each case in a hollow spiral-shaped manner in a shared imaginary lateral cylinder surface and the outer conductor being electrically insulated with respect to the second electrical conductor (227, 327, 327', 327", 327"', 427, 437, 527, 620).

2. A medical device according to any one of the preceding claims, wherein the functional conductor (26, 226, 326, 426, 526) is coupled over the longitudinal extension thereof, between the proximal end and the function electrode pole (22, 24, 30, 32, 222, 224, 322, 324, 422, 424), to a plurality of second electrical conductors (227, 327, 327', 327", 327"', 427, 437, 527, 620).

3. The medical device according to claim 2, wherein at least two of the second electrical conductors (227, 327, 327', 327", 327"', 427, 437, 527, 620) are coupled to each other in the form of an electrical series connection.

4. The medical device according to claim 2, wherein at least two of the second electrical conductors (227, 327, 327', 327", 327"', 427, 437, 527, 620) are coupled to each other in the form of an electrical parallel connection.

5. A medical device according to any one of the preceding claims, wherein the second electrical conductor (227, 327, 327', 327", 327"', 427, 437, 527, 620) is additionally coupled to the function electrode pole by way of a further coupling.

6. A medical device according to any one of the preceding claims, wherein the second electrical conductor is connected to a second electrode pole, which is different from the function electrode pole (22, 24, 30, 32, 222, 224, 322, 324, 422, 424).

7. The medical device according to claim 6, wherein the second electrode pole is covered with a dielectric layer.

8. A medical device according to any one of the preceding claims, wherein the second electrical conductor (227, 327, 327', 327", 327"', 427, 437, 527, 620) has a hollow spiral shape and surrounds the inner conductor of the functional conductor (26, 226, 326, 426, 526).

9. A medical device according to any one of the preceding claims, wherein the inner conductor of the functional conductor (26, 226, 326, 426, 526) is coupled to the second electrical conductor (227, 327, 327', 327", 327"', 427, 437, 527, 620) by way of at least one sliding contact.

## Revendications

1. Dispositif médical implantable temporaire ou permanent, avec
- au moins une première ligne électrique allongée (20) avec un conducteur fonctionnel (26, 226, 326, 426, 526) relié à un pôle d'électrode fonctionnelle (22, 24, 30, 32, 222, 224, 322, 324, 422, 424) pour la délivrance de signaux thérapeutiques ou pour la détection de signaux de diagnostic, et avec
- au moins un deuxième conducteur électrique (227, 327, 327', 327", 327"', 427, 437, 527, 620) accouplé au conducteur fonctionnel (26, 226, 326, 426, 526) et guidé avec lui dans une enveloppe isolante commune (533), dans lequel un accouplement entre le conducteur fonctionnel (26, 226, 326, 426, 526) et le deuxième conducteur électrique (227, 327, 327', 327", 327"', 427, 437, 527, 620) est formé pour intégrer au moins partiellement des ondes de fréquence radio électromagnétiques guidées dans le conducteur fonctionnel (26, 226, 326, 426, 526) dans le deuxième conducteur électrique (227, 327, 327', 327", 327"', 427, 437, 527, 620), dans lequel le conducteur fonctionnel (26, 226, 326, 426, 526) et le deuxième conducteur électrique (227, 327, 327', 327", 327"', 427, 437, 527, 620) sont accouplés par un accouplement ohmique, et **caractérisé en ce que** le conducteur fonctionnel comporte un conducteur intérieur et un conducteur extérieur, dans lequel, soit
- le deuxième conducteur électrique (227, 327, 327', 327", 327"', 427, 437, 527, 620) et un conducteur extérieur du conducteur fonctionnel (26, 226, 326, 426, 526) s'enroulent en forme de spirale sur une surface d'enveloppe de cylindre commune autour du conducteur intérieur du premier conducteur électrique, dans lequel le conducteur extérieur du conducteur fonctionnel (26, 226, 326, 426, 526) est électriquement isolé du deuxième conducteur électrique (227, 327, 327', 327", 327"', 427, 437, 527, 620), soit
- un conducteur extérieur du conducteur fonctionnel (26, 226, 326, 426, 526) s'enroule en forme de spirale autour du deuxième conducteur électrique (227, 327, 327', 327", 327"', 427, 437, 527, 620) et d'un conducteur intérieur du conducteur fonctionnel (26, 226, 326, 426, 526), dans lequel le conducteur intérieur et le deuxième conducteur électrique (227, 327, 327', 327", 327"', 427, 437, 527, 620) sont guidés respectivement en forme de spirale creuse sur une surface d'enveloppe de cylindre imaginaire commune, et le conducteur extérieur est électriquement isolé du deuxième conducteur électrique (227, 327, 327', 327", 327"', 427, 437, 527, 620).

2. Dispositif médical selon l'une des revendications précédentes, dans lequel le conducteur fonctionnel (26, 226, 326, 426, 526) est accouplé à plusieurs deuxièmes conducteurs électriques (227, 327, 327', 327", 327"', 427, 437, 527, 620) sur son étendue longitudinale entre son extrémité proximale et le pôle d'électrode fonctionnelle (22, 24, 30, 32, 222, 224, 322, 324, 422, 424).

3. Dispositif médical selon la revendication 2, dans lequel au moins deux des deuxièmes conducteurs électriques (227, 327, 327', 327", 327"', 427, 437, 527, 620) sont accouplés entre eux sous la forme d'un montage électrique en série.

4. Dispositif médical selon la revendication 2, dans lequel au moins deux des deuxièmes conducteurs électriques (227, 327, 327', 327", 327"', 427, 437, 527, 620) sont accouplés entre eux sous la forme d'un montage électrique en parallèle.

5. Dispositif médical selon l'une des revendications précédentes, dans lequel le deuxième conducteur électrique (227, 327, 327', 327", 327"', 427, 437, 527, 620) est en outre accouplé avec le pôle d'électrode fonctionnelle par un accouplement supplémentaire.

6. Dispositif médical selon l'une des revendications précédentes, dans lequel le deuxième conducteur électrique est relié à un deuxième pôle d'électrode différent du pôle d'électrode fonctionnelle (22, 24, 30, 32, 222, 224, 322, 324, 422, 424).

7. Dispositif médical selon la revendication 6, dans lequel le deuxième pôle d'électrode est recouvert d'une couche diélectrique.

8. Dispositif médical selon l'une des revendications précédentes, dans lequel le deuxième conducteur électrique (227, 327, 327', 327", 327"', 427, 437, 527, 620) présente une forme de spirale creuse et entoure le conducteur intérieur du conducteur fonctionnel (26, 226, 326, 426, 526).

9. Dispositif médical selon l'une des revendications précédentes, dans lequel le conducteur intérieur du conducteur fonctionnel (26, 226, 326, 426, 526) est accouplé au deuxième conducteur électrique (227, 327, 327', 327", 327"', 427, 437, 527, 620) par au moins un contact en boucle.
